(19) Europäisches Patentamt / European Patent Office / Office européen des brevets

(11) **EP 4 310 486 B1**

(12) **EUROPEAN PATENT SPECIFICATION**

(45) Date of publication and mention
of the grant of the patent:
**01.04.2026 Bulletin 2026/14**

(21) Application number: **22907679.9**

(22) Date of filing: **25.10.2022**

(51) International Patent Classification (IPC):
*G01N 33/00* (2006.01)    *G01N 25/66* (2006.01)
*G01K 1/02* (2021.01)    *G01D 7/00* (2006.01)
*H01M 4/04* (2006.01)    *H01M 10/42* (2006.01)

(52) Cooperative Patent Classification (CPC):
**G01N 25/66; G01N 33/0083; H01M 4/0402;**
**H01M 10/4285;** Y02E 60/10

(86) International application number:
**PCT/KR2022/016319**

(87) International publication number:
**WO 2023/113208 (22.06.2023 Gazette 2023/25)**

(54) **ABSOLUTE HUMIDITY CALCULATION APPARATUS AND OPERATION METHOD THEREOF**

VORRICHTUNG ZUR BERECHNUNG DER ABSOLUTEN FEUCHTIGKEIT
UNDBETRIEBSVERFAHREN DAFÜR

DISPOSITIF DE CALCUL D'HUMIDITÉ ABSOLUE ET PROCÉDÉ ASSOCIÉ DEFONCTIONNEMENT

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **14.12.2021 KR 20210179152**

(43) Date of publication of application:
**24.01.2024 Bulletin 2024/04**

(73) Proprietor: **LG ENERGY SOLUTION, LTD.**
**Seoul 07335 (KR)**

(72) Inventor: **PARK, Hyun Chul**
**Daejeon 34122 (KR)**

(74) Representative: **Plasseraud IP**
**104 Rue de Richelieu**
**CS92104**
**75080 Paris Cedex 02 (FR)**

(56) References cited:
JP-A- 2000 106 175    JP-A- 2012 083 206
JP-A- 2012 181 967    JP-A- 2021 188 962
KR-A- 20100 057 908    KR-B1- 100 314 166
KR-B1- 101 939 802    US-A1- 2010 212 590
US-A1- 2021 226 276

Description

## TECHNICAL FIELD

## CROSS-REFERENCE TO RELATED APPLICATION

The present application claims the benefit of the priority of Korean Patent Application No. 10-2021-0179152, filed on December 14, 2021.

## TECHNICAL FIELD

Embodiments disclosed herein relate to an absolute humidity calculation apparatus and an operation method therefor.

## BACKGROUND ART

Electric vehicles receive power from the outside to charge battery cells, and then motors are driven by the voltage charged in the battery cells to obtain power. A battery cell of an electric vehicle is manufactured by accommodating an electrode assembly in a battery case and injecting an electrolyte into the battery case.

The battery cell has high capacity and high energy density, and in order to maintain a long lifespan, an electrode active material applied on an electrode collector has to be dried during an electrode manufacturing process. However, when an electrode is not sufficiently dried or cracks occur on the electrode because the absolute humidity is not controlled during the electrode manufacturing process, the performance of the battery cell may deteriorate due to inappropriate reaction between electrodes. Also, degradation or explosion of the battery cell may occur.

JP2000106175A , JP2012083206A disclose method to measure humidity.

## DISCLOSURE OF THE INVENTION

## TECHNICAL PROBLEM

The purpose of embodiments disclosed herein is to provide
an absolute humidity calculation apparatus capable of accurately calculating absolute humidity in a battery manufacturing space in real time and an operation method therefor.

Technical problems of the embodiments disclosed herein are not limited to the above-mentioned technical problem, and other technical problems not mentioned above will be clearly understood by those skilled in the art from the descriptions below.

## TECHNICAL SOLUTION

An absolute humidity calculation apparatus is as disclosed in the attached set of claims. A first aspect of the invention relates to an absolute humidity calculation apparatus according to claim 1.

According to an embodiment, the first measurement unit may measure a temperature in a negative electrode-drying space for the battery.

According to an embodiment, the second measurement unit may measure the dew point data of exhaust air discharged from a negative electrode-drying space for the battery.

According to an embodiment, the controller may include a display that displays the absolute humidity.

According to a second aspect of the invention, a method for operating an absolute humidity calculation apparatus according to claim 5 includes measuring a temperature in a battery manufacturing space to acquire temperature data, measuring a dew point in the battery manufacturing space to acquire dew point data, and calculating absolute humidity in the battery manufacturing space on the basis of the temperature data and the dew point data.

According to an embodiment, the measuring of the temperature in the battery manufacturing space to acquire the temperature data may include measuring a temperature of a negative electrode-drying space for the battery.

According to an embodiment, the measuring of the dew point in the battery manufacturing space to acquire the dew point data may include acquiring the dew point data of exhaust air discharged from a negative electrode-drying space for the battery.

## ADVANTAGEOUS EFFECTS

According to an absolute humidity calculation apparatus and an operation method therefor according to an embodiment disclosed herein, absolute humidity in a battery manufacturing space may be accurately calculated in real time.

## BRIEF DESCRIPTION OF THE DRAWINGS

FIG. 1 is a view for illustrating an overall absolute humidity calculation apparatus according to an embodiment disclosed herein.
FIG. 2 is a block diagram for showing a configuration of the absolute humidity calculation apparatus according to an embodiment disclosed herein.
FIG. 3 is a flowchart for showing a method for operating the absolute humidity calculation apparatus according to an embodiment disclosed herein.
FIG. 4 is a block diagram of a hardware configuration of a computing system that implements the absolute humidity calculation apparatus according to an embodiment disclosed herein.

## MODE FOR CARRYING OUT THE INVENTION

Hereinafter, some embodiments disclosed herein will be described in detail with reference to the exemplary drawings. When reference numerals are given to elements in each drawing, it should be noted that the same elements will be designated by the same reference numerals if possible although they are shown in different drawings. Also, in describing embodiments disclosed herein, a detailed description of related known configurations or functions will be omitted when it is determined that the understanding of the embodiments disclosed herein is hindered by the detailed description.

In describing a component of embodiments disclosed herein, terms such as first, second, A, B, (a), and (c) may be used. These terms are only used to distinguish the component from other components, and the characteristics, orders, or sequences of the corresponding component are not limited by the terms. Also, unless otherwise defined, all terms used herein including technical or scientific terms have the same meanings as those generally understood by a person skilled in the art to which the embodiments disclosed herein pertain. Terms as defined in a commonly used dictionary should be construed as having the same meaning as in an associated technical context, and unless defined apparently herein, are not to be understood abnormally or as having an excessively formal meaning.

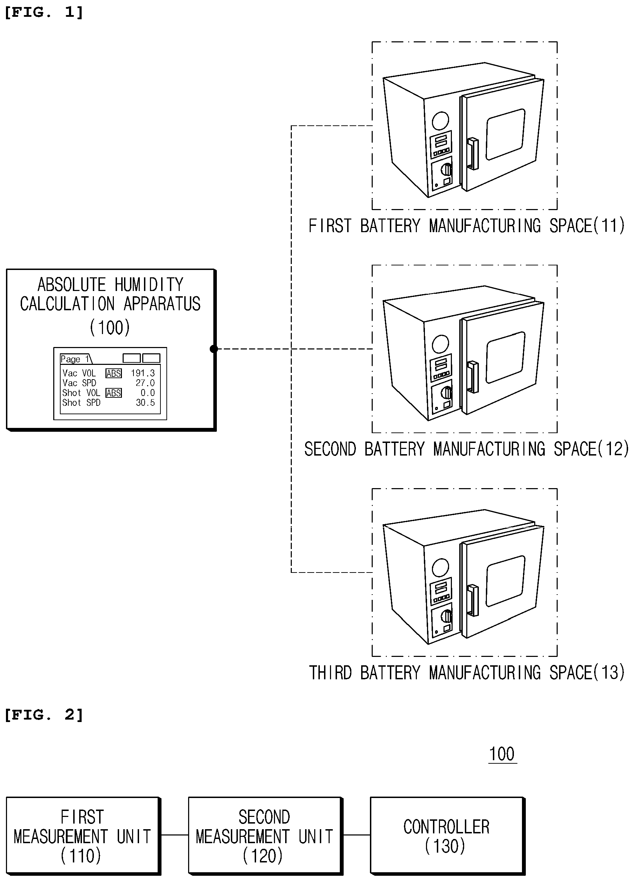

FIG. 1 is a view for illustrating an overall absolute humidity calculation apparatus according to an embodiment disclosed herein.

According to various embodiments, a battery may include a battery cell, which is a basic unit of the battery and is used by charging and discharging electrical energy. The battery cell may include, but not limited to, a lithium ion (Li-ion) battery, a lithium ion polymer (Li-ion polymer) battery, a nickel cadmium (Ni-Cd) battery, a nickel-metal hydride (Ni-MH) battery, and the like. The battery cell may supply power to a target device (not shown). To this end, the battery cell may be electrically connected to the target device. Here, target devices may include electrical, electronic, or mechanical devices, each of which is operated by receiving power from a battery pack (not shown) including a plurality of battery cells. For example, the target devices may include not only small products such as digital cameras, P-DVDs, MP3Ps, cellular phones, PDAs, portable game devices, power tools, and E-bikes, but also large products requiring high power such as electric vehicles and hybrid vehicles, and power storage devices or backup-power storage devices for storing surplus generated power and new renewable energy, but the embodiment is not limited thereto.

The battery cell may include an electrode assembly, a battery case accommodating the electrode assembly therein, and an electrolyte injected into the battery case to activate the electrode assembly. The electrode assembly is formed by interposing a separator between a positive electrode formed by coating a positive electrode collector with a positive electrode active material and a negative electrode formed by coating a negative electrode collector with a negative electrode active material. Depending on the type of battery case, the electrode assembly may be manufactured in a jelly roll type, a stack type, or the like and accommodated inside the battery case.

According to an embodiment, the battery cell may be manufactured through a series of manufacturing processes that include an electrode process, an assembly process, and a formation process. Here, the electrode process may include a process in which a positive electrode and a negative electrode are manufactured by attaching a positive electrode material and a negative electrode material to a positive electrode collector and a negative electrode collector, respectively. Specifically, the electrode process may include a mixing process, a coating process, a press process, and a slitting process.

During the coating process, slurry containing a positive electrode active material is applied to the surface of the positive electrode collector, and slurry containing a negative electrode active material is applied to the surface of the negative electrode collector. In the slurry applied to the negative electrode collector, water is typically used as a solvent. Here, the slurry is provided in a fluid state containing a large amount of solvent. Also, the solvent has to be removed after the slurry is applied to the collector so as not to damage an active material layer. Accordingly, the coating process includes a drying process in which the electrode active material applied on the collector is dried to remove the solvent and moisture in the slurry.

For example, during the drying process, an electrode drying oven may be used to dry the electrode active material applied on the collector. During the drying process, the absolute humidity in the electrode drying oven has to be controlled to sufficiently dry the electrode, and the occurrence of cracks in the electrode has to be prevented.

When cracks occur during the drying process, coating defects may occur on the electrode. Therefore, when assembling an electrode assembly or using a battery cell, an organic/inorganic composite porous layer may be easily detached. This may deteriorate the safety of the battery cell. In addition, it is difficult to identify micro-cracks with measurement equipment or naked eyes, and thus, the cracks are discovered after the final battery cell is produced. In this case, the battery cell cannot be used in a product, and thus, the yield of processes is deteriorated.

Hereinafter, an electrode drying process will be described as an example of the battery process.

FIG. 1 is a view for illustrating an overall absolute humidity calculation apparatus according to an embodiment disclosed herein.

Referring to FIG. 1, a battery manufacturing space 10 may include a first battery manufacturing space 11, a second battery manufacturing space 12, and a third battery manufacturing space 13. FIG. 1 illustrates that a battery manufacturing space 10 has three spaces, but the embodiment is not limited thereto. The battery manufacturing space 10 may have n apparatuses (n is a natural number of 3 or more).

An absolute humidity calculation apparatus 100 may collect data of pieces of equipment operated in a battery process in real time and deliver the data to a user (e.g., administrator). The absolute humidity calculation apparatus 100 may display the data on a screen in real time. For example, the absolute humidity calculation apparatus 100 may collect in real time the data of the pieces of equipment, which are operated in the first battery manufacturing space 11, the second battery manufacturing space 12, and the third battery manufacturing space 13, and may display the data on the screen.

Here, each of the first battery manufacturing space 11, the second battery manufacturing space 12, and the third battery manufacturing space 13 may include an electrode drying ovens for drying a solvent of slurry applied to an electrode collector. Each of the first battery manufacturing space 11, the second battery manufacturing space 12, and the third battery manufacturing space 13 may include a vent member (not shown) for discharging air containing moisture to the outside, and the moisture evaporated from the surface of the electrode may be discharged via the vent member.

Each of the vent members of the first battery manufacturing space 11, the second battery manufacturing space 12, and the third battery manufacturing space 13 may include a vent duct for discharging air to the outside and a vent pump for generating a suction force to forcibly discharge the air to the outside via the vent duct.

The absolute humidity calculation apparatus 100 may collect and analyze in real time the data of the pieces of equipment, which are operated in the first battery manufacturing space 11, the second battery manufacturing space 12, and the third battery manufacturing space 13. For example, the absolute humidity calculation apparatus 100 may collect data or graph data generated in the first battery manufacturing space 11, such as the temperature, relative humidity, pressure, process progress status, presence or absence of alarms, and quantity of the first battery manufacturing space 11, from pieces of equipment operated in the first battery manufacturing space 11, and may analyze the data.

FIG. 2 is a block diagram for showing a configuration of the absolute humidity calculation apparatus according to an embodiment disclosed herein.

Hereinafter, the configuration of the absolute humidity calculation apparatus 100 will be described in detail with reference to FIG. 2. Also, among the first battery manufacturing space 11, the second battery manufacturing space 12, and the third battery manufacturing space 13 of the battery manufacturing space 10, the first battery manufacturing space 11 will be described below as an example. However, the description may also be applied, in substantially the same manner, to the second battery manufacturing space 12 and the third battery manufacturing space 13.

Referring to FIG. 2, the absolute humidity calculation apparatus 100 may include a first measurement unit 110, a second measurement unit 120, and a controller 130.

The first measurement unit 110 may measure a temperature in the first battery manufacturing space 11 and acquire temperature data. For example, the first measurement unit 110 may be located in a negative electrode-drying space for a battery and measure the temperature of the negative electrode-drying space for the battery.

The first measurement unit 110 may transmit temperature data, which has been acquired by measuring the temperature in the first battery manufacturing space 11, to the controller 130 in the form of an electrical signal.

The second measurement unit 120 may measure a dew point in the first battery manufacturing space 11 and acquire dew point data. Here, the dew point is the temperature at which water vapor begins to condense as the temperature of the atmosphere decreases, and may be defined as the temperature at which the pressure of water vapor becomes equal to the saturation vapor pressure. For example, the second measurement unit 120 may be located in the negative electrode-drying space for the battery and acquire the dew point data of exhaust air discharged from the negative electrode-drying space for the battery.

According to an embodiment, the second measurement unit 120 may be located in the vent duct that discharges air containing moisture in the first battery manufacturing space 11 to the outside. The second measurement unit 120 may acquire the dew point data in the first battery manufacturing space 11 by cooling the temperature of the exhaust air in the first battery manufacturing space 11. That is, the second measurement unit 120 may lower the temperature of the exhaust air in the first battery manufacturing space 11 by driving a built-in cooler or by driving a cooler included in the vent member of the first battery manufacturing space 11, thereby acquiring the dew point data in the first battery manufacturing space 11.

The second measurement unit 120 may transmit the dew point data, which has been acquired by lowering the temperature of the exhaust air in the first battery manufacturing space 11, to the controller 130 in the form of an electrical signal.

The controller 130 may include an absolute humidity calculation algorithm that calculates absolute humidity on the basis of the temperature data and the vapor pressure data. For example, the controller 130 may calculate the absolute humidity in the first battery manufacturing space 11, on the basis of the temperature data in the first battery manufacturing space 11 acquired from the first measurement unit 110 and the dew point data in the first battery manufacturing space 11 acquired from the second measurement unit 120.

Specifically, regarding a humidity measurement method, the absolute humidity calculation algorithm of the controller 130 may use a method for calculating the absolute humidity on the basis of the dew point data in the corresponding battery manufacturing space 10, instead of using a method for performing conversion to the absolute humidity on the basis of the relative humidity therein. The exhaust air generated from an electrode drying oven that dries the electrode of the battery is in a superheated state of high temperature of 130°C to 150°C or higher, and the amount of saturated water vapor is abnormally high. Therefore, since the relative humidity is measured as a very low value, the absolute humidity may not be measured or a significant error may occur. On the other hand, when the dew point data is acquired by cooling the high-temperature exhaust air, the absolute humidity changes at a constant rate according to the temperature at the same dew point. Accordingly, the absolute humidity of the battery manufacturing space 10 may be calculated on the basis of the dew point data and the temperature data.

The controller 130 calculates the absolute humidity as the temperature data and the vapor pressure data are input to an absolute humidity conversion formula. Equation (1) below represents the absolute humidity conversion formula.

Equation (1)

$$A = C * P_w / T \qquad \qquad \cdots \cdots (1)$$

In Equation (1), A represents the absolute humidity, and the unit is $g/m^3$. C is a constant value and has a value of 216.679 gK/J. $P_w$ is a value of vapor pressure, and the unit is hPa. $P_w$ is acquired on the basis of the dew point data that is data that does not change with temperature change at the same dew point. T is a value of absolute temperature, and the unit is K.

Equation (1) requires a total of three values of C, $P_w$, and T, but C is a fixed constant value, and $P_w$ is acquired on the basis of the dew point data. Therefore, in Equation (1) that is the absolute humidity conversion formula, only T data is used as a parameter that affects the absolute humidity.

The controller 130 calculates the absolute humidity A in the first battery manufacturing space 11, as the temperature data T in the first battery manufacturing space 11 acquired from the first measurement unit 110 and the vapor pressure data $P_w$ acquired on the basis of the dew point data in the first battery manufacturing space 11 acquired from the second measurement unit 120 are input to Equation (1).

According to an embodiment, the controller 130 may include a display for displaying the absolute humidity. The controller 130 may calculate the absolute humidity in at least one of the first battery manufacturing space 11, the second battery manufacturing space 12, and the third battery manufacturing space 13, and display the absolute humidity on a display.

As described above, in the absolute humidity calculation apparatus 100 according to an embodiment disclosed herein, the absolute humidity in the battery manufacturing space may be accurately calculated in real time.

In addition, since the absolute humidity calculation apparatus 100 calculates the absolute humidity by acquiring the data from the first measurement unit 110 and the second measurement unit 120 in real time without being provided with or connected to a separate device, the absolute humidity during the battery manufacturing process may be rapidly controlled. Therefore, the absolute humidity analysis cost and absolute humidity analysis time may be reduced.

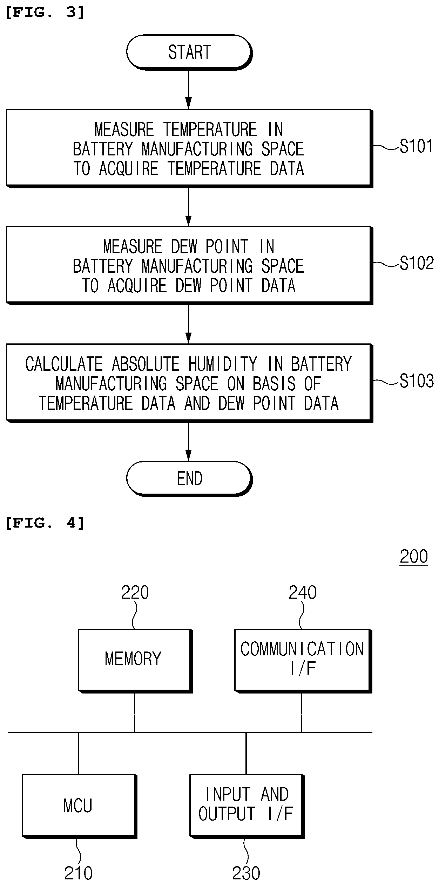

FIG. 3 is a flowchart for showing a method for operating the absolute humidity calculation apparatus according to an embodiment disclosed herein.

Hereinafter, a method for operating an absolute humidity calculation apparatus 100 will be described with reference to FIGS. 1 and 2.

The absolute humidity calculation apparatus 100 may be substantially the same as the absolute humidity calculation apparatus 100 described with reference to FIGS. 1 and 2, and thus, this apparatus will be briefly described below to avoid duplication of description.

Referring to FIG. 3, the method for operating the absolute humidity calculation apparatus 100 may include measuring a temperature in a battery manufacturing space to acquire temperature data (S101), measuring a dew point in the battery manufacturing space to acquire dew point data (S102), and calculating absolute humidity in the battery manufacturing space on the basis of the temperature data and the dew point data (S103).

In operation S101, a first measurement unit 110 may measure the temperature in a first battery manufacturing space 11 and acquire the temperature data. For example, in operation S101, the first measurement unit 110 may be located in a negative electrode-drying space for a battery and measure the temperature of the negative electrode-drying space for the battery.

In operation S101, the first measurement unit 110 may transmit the temperature data, which has been acquired by measuring the temperature in the first battery manufacturing space 11, to a controller 130 in the form of an electrical signal.

In operation S102, a second measurement unit 120 may measure the dew point in the first battery manufacturing space 11 and acquire the dew point data. For example, in operation S102, the second measurement unit 120 may acquire the dew point data in the first battery manufacturing space 11 by cooling the temperature of exhaust air in the first battery manufacturing space 11. That is, in operation S102, the second measurement unit 120 may lower the temperature of the exhaust air in the first battery manufacturing space 11 by driving a built-in cooler or by driving a cooler included in a vent member of the first battery manufacturing space 11, thereby acquiring the dew point data in the first battery manufacturing space 11.

In operation S102, the second measurement unit 120 may transmit the dew point data, which has been acquired by lowering the temperature of the exhaust air in the first battery manufacturing space 11, to the controller 130 in the form of an electrical signal.

In operation S103, the controller 130 may calculate the absolute humidity in the battery manufacturing space on the basis of the temperature data and the dew point data. For example, in operation S103, the controller 130 may calculate the absolute humidity in the first battery manufacturing space 11, on the basis of the temperature data in the first battery manufacturing space 11 acquired from the first measurement unit 110 and the dew point data in the first battery manufacturing space 11 acquired from the second measurement unit 120.

In operation S103, the controller 130 may calculate the absolute humidity as the temperature data and vapor pressure data are input to an absolute humidity conversion formula. In operation S103, the controller 130 may acquire the vapor pressure data in the first battery manufacturing space 11 on the basis of the dew point data. For example, in operation S103, the controller 130 may calculate the absolute humidity in the first battery manufacturing space 11, as the temperature data in the first battery manufacturing space 11 acquired from the first measurement unit 110 and the vapor pressure data acquired on the basis of the dew point data in the first battery manufacturing space 11 acquired from the second measurement unit 120 are input to the absolute humidity conversion formula.

In operation S103, the controller 130 may calculate the absolute humidity in at least one of the first battery manufacturing space 11, a second battery manufacturing space 12, and a third battery manufacturing space 13, and display the absolute humidity on a display.

FIG. 4 is a block diagram of a hardware configuration of a computing system that implements the absolute humidity calculation apparatus according to an embodiment disclosed herein.

Referring to FIG. 4, a computing system 200 according to an embodiment disclosed herein may include an MCU 210, a memory 220, an input and output I/F 230, and a communication I/F 240.

The MCU 210 may include a processor for executing various programs (e.g., a program for converting the absolute humidity of an absolute humidity calculation apparatus 100) stored in the memory 220, processing various data of these programs, and performing functions of the absolute humidity calculation apparatus 100 shown in FIG. 1 illustrated above.

The memory 220 may store various programs related to the operation of the absolute humidity calculation apparatus 100. Also, the memory 220 may store operation data of the absolute humidity calculation apparatus 100.

A plurality of memories 220 may be provided as necessary. The memory 220 may include a volatile memory or a non-volatile memory. The memory 220 as the volatile memory may use RAM, DRAM, SRAM, or the like. The memory 220 as the non-volatile memory may use ROM, PROM, EAROM, EPROM, EEPROM, flash memory, or the like. The memories 220 listed above are merely examples, and the embodiment is not limited to these examples.

The input and output I/F 230 may provide an interface that connects an input device (not shown) such as a keyboard, mouse, or touch panel, an output device such as a display (not shown), and the MCU 210 so as to transmit and receive data.

The communication I/F 240 may include various devices which are configured to transmit and receive various data to and from a server and can support wired or wireless communication. For example, programs or various data for resistance measurement and abnormality diagnosis may be transmitted to and received from a separately provided external server via the communication I/F 240.

As described above, the computer program according to an embodiment disclosed herein is recorded in the memory 220 and processed by the MCU 210, and thus may be embodied, for example, as a module that performs each function of the absolute humidity calculation apparatus 100 described with reference to FIGS. 1 and 2.

The technical ideas of the present disclosure have been described merely for illustrative purposes, and those skilled in the art, to which the present disclosure pertains, will appreciate that various changes and modifications are possible without departing from the scope of the invention which is defined by the appended claims.

Therefore, the scope of the present invention is not limited by the embodiments disclosed herein. The protective scope of the present invention is defined by the appended claims.

**Claims**

1. An absolute humidity calculation apparatus comprising (100):

   a first measurement unit (110) configured to measure a temperature in a battery manufacturing space (11, 12, 13) and acquire temperature data;
   a second measurement unit (120) configured to measure a dew point in the battery manufacturing space (11, 12, 13)and acquire dew point data;
   a controller (130) configured to calculate absolute humidity in the battery manufacturing space (11, 12, 13) on the basis of the temperature data and the dew point data,
   wherein the manufacturing space (11, 12, 13) comprises an electrode drying oven,
   wherein the controller (130) calculates the absolute humidity as the temperature data and the vapor pressure data are input to an absolute humidity conversion formula of Equation (1) below

   $$A = C * Pw / T,$$

   A represents the absolute humidity, and the unit is $g/m^3$, C is a constant value and has a value of 216.679 gK/J, $P_w$ is a value of vapor pressure in the battery manufacturing space, and the unit is hPa, Pw is acquired on the basis of the dew point data that is data that does not change with temperature change at the same dew point, T is a value of absolute temperature, and the unit is K..

2. The absolute humidity calculation apparatus (100) of claim 1, wherein the first measurement unit (110) measures a temperature in a negative electrode-drying space for the battery.

3. The absolute humidity calculation apparatus (100) of claim 1, wherein the second measurement unit (120) measures the dew point data of exhaust air discharged from a negative electrode-drying space for the battery.

4. The absolute humidity calculation apparatus (100) of claim 1, wherein the controller (130) comprises a display that displays the absolute humidity.

5. A method for operating an absolute humidity calculation apparatus (100) according to claim 1, the method comprising:

   measuring a temperature in a battery manufacturing space (11, 12, 13) to acquire temperature data;
   measuring a dew point in the battery manufacturing space (11, 12, 13) to acquire dew point data; and
   calculating absolute humidity in the battery manufacturing space (11, 12, 13) on the basis of the temperature data and the dew point data.

6. The method of claim 5, wherein the measuring of the temperature in the battery manufacturing space (11, 12, 13) to acquire the temperature data comprises measuring a temperature of a negative electrode-drying space for the battery.

7. The method of claim 5, wherein the measuring of the dew point in the battery manufacturing space (11, 12, 13) to acquire the dew point data comprises acquiring the dew point data of exhaust air discharged from a negative electrode-drying space for the battery.

**Patentansprüche**

1. Vorrichtung zum Berechnen einer absoluten Feuchtigkeit (100), umfassend:

   eine erste Messeinheit (110), welche dazu eingerichtet ist, eine Temperatur in einem Batterie-Herstellungsraum (11, 12, 13) zu messen und Temperaturdaten aufzunehmen;
   eine zweite Messeinheit (120), welche dazu eingerichtet ist, einen Taupunkt in dem Batterie-Herstellungsraum (11, 12, 13) zu messen und Taupunktdaten aufzunehmen;
   eine Steuereinheit (130), welche dazu eingerichtet ist, eine absolute Feuchtigkeit in dem Batterie-Herstellungsraum (11, 12, 13) auf der Grundlage der Temperaturdaten und der Taupunktdaten zu berechnen,
   wobei der Herstellungsraum (11, 12, 13) einen Elektroden-Trocknungsofen umfasst,
   wobei die Steuereinheit (130) die absolute Feuchtigkeit berechnet, wenn die Temperaturdaten und die Dampfdruckdaten in eine Umrechnungsformel der absoluten Feuchtigkeit der Gleichung (1) unten eingegeben werden

$$\text{Gleichung (1):}$$

$$A = C^* P_w / T,$$

   wobei A die absolute Feuchtigkeit repräsentiert und die Einheit $g/m^3$ ist, C ein konstanter Wert ist und einen Wert von 216,679 gK/J aufweist, $P_w$ ein Wert eines Dampfdrucks in dem Batterie-Herstellungsraum ist und die Einheit hPa ist, wobei $P_w$ auf der Grundlage der Taupunktdaten aufgenommen wird, welche Daten sind, welche sich nicht mit einer Temperaturänderung an demselben Taupunkt ändern, T ein Wert einer absoluten Temperatur ist und die Einheit K ist.

2. Vorrichtung zum Berechnen einer absoluten Feuchtigkeit (100) nach Anspruch 1, wobei die erste Messeinheit (110) eine Temperatur in einem Negativelektroden-Trocknungsraum für die Batterie misst.

3. Vorrichtung zum Berechnen einer absoluten Feuchtigkeit (100) nach Anspruch 1, wobei die zweite Messeinheit (120) die Taupunktdaten von Abgasluft misst, welche von einem Negativelektroden-Trocknungsraum für die Batterie abgegeben wird.

4. Vorrichtung zum Berechnen einer absoluten Feuchtigkeit (100) nach Anspruch 1, wobei die Steuereinheit (130) eine Anzeige umfasst, welche die absolute Feuchtigkeit anzeigt.

5. Verfahren zum Betreiben einer Vorrichtung zum Berechnen einer absoluten Feuchtigkeit (100) nach Anspruch 1, das Verfahren umfassend:

   Messen einer Temperatur in einem Batterie-Herstellungsraum (11, 12, 13), um Temperaturdaten aufzunehmen;
   Messen eines Taupunkts in dem Batterie-Herstellungsraum (11, 12, 13), um Taupunktdaten aufzunehmen; und
   Berechnen einer absoluten Feuchtigkeit in dem Batterie-Herstellungsraum (11, 12, 13) auf der Grundlage der Temperaturdaten und der Taupunktdaten.

6. Verfahren nach Anspruch 5, wobei das Messen der Temperatur in dem Batterie-Herstellungsraum (11, 12, 13) zum Aufnehmen der Temperaturdaten ein Messen einer Temperatur eines Negativelektroden-Trocknungsraums für die Batterie umfasst.

7. Verfahren nach Anspruch 5, wobei das Messen des Taupunkts in dem Batterie-Herstellungsraum (11, 12, 13) zum Aufnehmen der Taupunktdaten ein Aufnehmen der Taupunktdaten von Abgasluft umfasst, welche von einem Negativelektroden-Trocknungsraum für die Batterie abgegeben wird.

**Revendications**

1. Appareil de calcul d'humidité absolue (100) comprenant :

une première unité de mesure (110) configurée pour mesurer une température dans un espace de fabrication (11, 12, 13) de batteries et acquérir des données de température ;

une seconde unité de mesure (120) configurée pour mesurer un point de rosée dans l'espace de fabrication (11, 12, 13) de batteries et acquérir des données de point de rosée ;

un dispositif de commande (130) configuré pour calculer l'humidité absolue dans l'espace de fabrication (11, 12, 13) de batteries sur la base des données de température et des données de point de rosée,

dans lequel l'espace de fabrication (11, 12, 13) comprend une étuve de séchage d'électrodes,

dans lequel le dispositif de commande (130) calcule l'humidité absolue lorsque les données de température et les données de pression de vapeur sont entrées dans une formule de conversion d'humidité absolue de l'Équation (1) ci-dessous

$$\text{Équation (1) :}$$

$$A = C*Pw/T,$$

A représente l'humidité absolue, et l'unité est le g/m$^3$, C est une valeur constante et a une valeur de 216,679 gK/J, $P_w$ est une valeur de pression de vapeur dans l'espace de fabrication de batteries, et l'unité est le hPa, Pw est acquise sur la base des données de point de rosée qui sont des données qui ne changent pas avec un changement de température au même point de rosée, T est une valeur de température absolue, et l'unité est le K.

2. Appareil de calcul d'humidité absolue (100) selon la revendication 1, dans lequel la première unité de mesure (110) mesure une température dans un espace de séchage d'électrodes négatives pour la batterie.

3. Appareil de calcul d'humidité absolue (100) selon la revendication 1, dans lequel la seconde unité de mesure (120) mesure les données de point de rosée de l'air d'échappement évacué d'un espace de séchage d'électrodes négatives pour la batterie.

4. Appareil de calcul d'humidité absolue (100) selon la revendication 1, dans lequel le dispositif de commande (130) comprend un afficheur qui affiche l'humidité absolue.

5. Procédé de fonctionnement d'un appareil de calcul d'humidité absolue (100) selon la revendication 1, le procédé comprenant :

la mesure d'une température dans un espace de fabrication (11, 12, 13) de batteries pour acquérir des données de température ;

la mesure d'un point de rosée dans l'espace de fabrication (11, 12, 13) de batteries pour acquérir des données de point de rosée ; et

le calcul de l'humidité absolue dans l'espace de fabrication (11, 12, 13) de batteries sur la base des données de température et des données de point de rosée.

6. Procédé selon la revendication 5, dans lequel la mesure de la température dans l'espace de fabrication (11, 12, 13) de batteries pour acquérir les données de température comprend la mesure d'une température d'un espace de séchage d'électrodes négatives pour la batterie.

7. Procédé selon la revendication 5, dans lequel la mesure du point de rosée dans l'espace de fabrication (11, 12, 13) de batteries pour acquérir les données de point de rosée comprend l'acquisition des données de point de rosée de l'air d'échappement évacué d'un espace de séchage d'électrodes négatives pour la batterie.

[FIG. 1]

ABSOLUTE HUMIDITY
CALCULATION APPARATUS
(100)

| Page 1 | | |
|---|---|---|
| Vac VOL | ABS | 191.3 |
| Vac SPD | | 27.0 |
| Shot VOL | ABS | 0.0 |
| Shot SPD | | 30.5 |

FIRST BATTERY MANUFACTURING SPACE(11)

SECOND BATTERY MANUFACTURING SPACE(12)

THIRD BATTERY MANUFACTURING SPACE(13)

[FIG. 2]

100

| FIRST MEASUREMENT UNIT (110) | SECOND MEASUREMENT UNIT (120) | CONTROLLER (130) |
|---|---|---|

[FIG. 3]

```
         ┌─────────────┐
         │    START    │
         └─────────────┘
                │
                ▼
   ┌────────────────────────────┐
   │    MEASURE TEMPERATURE IN   │
   │ BATTERY MANUFACTURING SPACE │─── S101
   │  TO ACQUIRE TEMPERATURE DATA│
   └────────────────────────────┘
                │
                ▼
   ┌────────────────────────────┐
   │    MEASURE DEW POINT IN     │
   │ BATTERY MANUFACTURING SPACE │─── S102
   │  TO ACQUIRE DEW POINT DATA  │
   └────────────────────────────┘
                │
                ▼
   ┌──────────────────────────────────┐
   │ CALCULATE ABSOLUTE HUMIDITY IN    │
   │ BATTERY MANUFACTURING SPACE ON    │─── S103
   │ BASIS OF TEMPERATURE DATA AND     │
   │ DEW POINT DATA                    │
   └──────────────────────────────────┘
                │
                ▼
         ┌─────────────┐
         │     END     │
         └─────────────┘
```

[FIG. 4]

200

```
      220                    240
       │                      │
  ┌──────────┐         ┌──────────────┐
  │  MEMORY  │         │ COMMUNICATION │
  │          │         │     I/F       │
  └──────────┘         └──────────────┘
       │                      │
  ─────┼──────────────────────┼─────
       │                      │
  ┌──────────┐         ┌──────────────┐
  │   MCU    │         │  INPUT AND    │
  │          │         │  OUTPUT I/F   │
  └──────────┘         └──────────────┘
       │                      │
      210                    230
```

**EP 4 310 486 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210179152
- JP 2000106175 A
- JP 2012083206 A